# EUROPÄISCHE PATENTSCHRIFT

(11) **EP 1 439 829 B1**
(45) Veröffentlichungstag und Bekanntmachung des Hinweises auf die Patenterteilung: **07.09.2005**
(21) Anmeldenummer: 02781278.3
(22) Anmeldetag: 22.10.2002
(51) Int. Cl.: A61K 31/135, A61K 9/22

(54) **3-(3-DIMETHYLAMINO-1-ETHYL-2-METHYL-PROPYL)PHENOL ENTHALTENDES ARZNEIMITTEL MIT VERZÖGERTER WIRKSTOFFFREISETZUNG**
PHARMACEUTICAL CONTAINING 3-(3-DIMETHYLAMINO-1-ETHYL-2-METHYL-PROPYL)PHENOL AND PROVIDING DELAYED RELEASE OF THE ACTIVE INGREDIENT
MEDICAMENT CONTENANT DU 3-(3-DIMETHYLAMINO-1-ETHYL-2-METHYL-PROPYL)PHENOL, CARACTERISE PAR UNE LIBERATION DIFFEREE DU PRINCIPE ACTIF

(30) Priorität: 24.10.2001 DE 10152469; 16.10.2002 DE 10248309
(43) Veröffentlichungstag der Anmeldung: 28.07.2004
(73) Patentinhaber: Grünenthal GmbH, 52078 Aachen (DE)
(72) Erfinder: BARTHOLOMÄUS, Johannes, 52080 Aachen (DE); ZIEGLER, Iris, 52159 Rott-Roetgen (DE)
(86) Internationale Anmeldenummer: PCT/EP2002/011809
(87) Internationale Veröffentlichungsnummer: WO 2003/035053

(56) Entgegenhaltungen:
- EP-A- 0 693 475

## Beschreibung

Die Erfindung betrifft eine pharmazeutische Formulierung mit verzögerter Wirkstofffreisetzung, die 3-(3-Dimethylamino-1-ethyl-2-methylpropyl)phenol oder eines seiner pharmzeutisch akzeptablen Salze in einer Matrix enthält.

3-(3-Dimethylamino-1-ethyl-2-methyl-propyl)phenol ist aus der EP 0 693 475 B1 als ein analgetisch wirksames Arzneimittel bekannt und kann oral appliziert werden. Die üblichen Formulierungen für die orale Verabreichung von 3-(3-Dimethylamino-1-ethyl-2-methyl-propyl)phenol führen zu einer raschen Freisetzung des Wirkstoffs im Gastrointestinaltrakt, so daß seine analgetische Wirkung schnell einsetzt. Zugleich beobachtet man ein rasches Abklingen der Wirkung. Somit erfordert die Behandlung starker chronischer Schmerzen mit 3-(3-Dimethylamino-1-ethyl-2-methylpropyl)phenol bislang die Verabreichung des Arzneimittels in relativ kurzen Abständen, beispielsweise vier- bis sechsmal täglich, um so eine ausreichende Wirkstoffkonzentration im Blutplasma des Patienten zu gewährleisten. Die Notwendigkeit einer häufigen Dosierung führt jedoch leicht zu Fehlern bei der Einnahme sowie zu unerwünschten Plasmakonzentrationsschwankungen, was der Patientencompliance und dem therapeutischen Nutzen abträglich ist, insbesondere bei der Behandlung chronischer Schmerzzustände. Eine pharmazeutische Darreichungsform mit verzögerter Freisetzung (Retardformulierung) für die orale Applikation des Wirkstoffs 3-(3-Dimethylamino-1-ethyl-2-methylpropyl)phenol ist daher wünschenswert.

Im Stand der Technik sind allgemein Retardformulierungen für eine große Zahl verschiedener Wirkstoffe bekannt. Übliche Retardierungsformen sind u.a. Überzugsretardierungen und Matrixretardierungen.

Im Falle von Überzugsretardierungen, wie sie z.B. in der DE 36 25 458 A1 beschrieben sind, wird der einen Wirkstoff enthaltende Kern einer pharmazeutischen Zusammensetzung mit einem die Freisetzung des Wirkstoffs verzögernden Überzug aus einem oder mehreren hydrophilen und/oder hydrophoben Polymeren versehen.

Bei Matrixretardierungen ist der Wirkstoff in einer aus einem oder mehreren Trägermaterialien gebildeten Matrix enthalten, welche die Freisetzung des Wirkstoffs steuert. So offenbart beispielsweise die DE 33 09 516 A1 ein Verfahren zur Herstellung von Matrixformulierungen mit Hydroxypropylmethylcellulose (HPMC) als Trägermaterial und zum Teil verzögerter Freisetzung des Wirkstoffs, wobei das Trägermaterial nicht mehr als ein Drittel des Gewichts der Formulierung ausmacht und aus mindestens einer Hydroxypropylmethylcellulose besteht, die einen Methoxygehalt von 16-24 Gew.-%, einen Hydroxypropylgehalt von 4-32 Gew.-% und ein zahlenmäßig durchschnittliches Molekulargewicht von mindestens 50.000 aufweist. Die in der DE 33 09 516 A1 offenbarten Formulierungen enthalten HPMCs mit Viskositäten (in 2 gew.-%iger wäßriger Lösung bei 20 °C) zwischen 15 und 30.000 cPs (15 bis 30.000 mPa•s). Ein vom pH-Wert des Auflösungsmediums unabhängiges Freisetzungsverhalten wird in der DE 33 09 516 A1 nicht offenbart.

Es ist somit eine Aufgabe der vorliegenden Erfindung, eine 3-(3-Dimethylamino-1-ethyl-2-methyl-propyl)phenol enthaltende pharmazeutische Formulierung mit verzögerter Wirkstofffreisetzung bereitzustellen.

Gelöst wird diese Aufgabe durch eine pharmazeutische Formulierung mit verzögerter Freisetzung, die 3-(3-Dimethylamino-1-ethyl-2-methylpropyl)phenol oder ein pharmazeutisch annehmbares Salz davon in einer Matrix mit verzögerter Wirkstofffreisetzung enthält,
wobei die Matrix 1 bis 80 Gew.-%, bevorzugt 5 bis 80 Gew.-%, eines oder mehrerer hydrophiler oder hydrophober Polymere als pharmazeutisch annehmbaren Matrixbildner enthält und in vitro die folgende Freisetzungsgeschwindigkeit aufweist, gemessen unter Anwendung der Ph. Eur. Paddle Method bei 75 U/min in einem Puffer (gemäß Ph. Eur.) bei einem pH-Wert von 6,8 bei 37 °C und unter UV-spektrometrischer Detektion:
3-35 Gew.-% 3-(3-Dimethylamino-1-ethyl-2-methyl-propyl)phenol (bezogen auf 100 Gew.-% Wirkstoff) nach 0,5 Stunden freigesetzt,
5-50 Gew.-% 3-(3-Dimethylamino-1-ethyl-2-methyl-propyl)phenol nach 1 Stunde freigesetzt,
10-75 Gew.-% 3-(3-Dimethylamino-1-ethyl-2-methyl-propyl)phenol nach 2 Stunden freigesetzt,
15-82 Gew.-% 3-(3-Dimethylamino-1-ethyl-2-methyl-propyl)phenol nach 3 Stunden freigesetzt,
30-97 Gew.-% 3-(3-Dimethylamino-1-ethyl-2-methyl-propyl)phenol nach 6 Stunden freigesetzt,
mehr als 50 Gew.-% 3-(3-Dimethylamino-1-ethyl-2-methylpropyl)phenol nach 12 Stunden freigesetzt,
mehr als 70 Gew.-% 3-(3-Dimethylamino-1-ethyl-2-methylpropyl)phenol nach 18 Stunden freigesetzt,
mehr als 80 Gew.-% 3-(3-Dimethylamino-1-ethyl-2-methylpropyl)phenol nach 24 Stunden freigesetzt.

Es hat sich überraschend gezeigt, daß die erfindungsgemäße Formulierung den Wirkstoff 3-(3-Dimethylamino-1-ethyl-2-methyl-propyl)phenol bei oraler Verabreichung verzögert freisetzt und sich somit für eine Verabreichung im Abstand von mindestens 12 Stunden eignet. Die erfindungsgemäße Formulierung erlaubt demnach eine Schmerztherapie, in deren Verlauf das Analgetikum 3-(3-Dimethylamino-1-ethyl-2-methyl-propyl)phenol nur noch einmal täglich, z.B. im Abstand von 24 h, oder zweimal täglich, vorzugsweise im Abstand von 12 Stunden, verabreicht werden muß, um eine ausreichende Plasmakonzentration des Wirkstoffs zu gewährleisten. Eine entsprechende Wirkdauer und die Aufrechterhaltung ausreichender Blutplasma-Spiegel wird durch Simulationsstudien und experimentelle Untersuchungen belegt.

Besonders überraschend ist dabei, daß die erfindungsgemäße Formulierung nicht nur aufgrund der verzögerten Freisetzung eine langandauerde therapeutische Wirksamkeit über einen relativ langen Zeitraum (mindestens 12 Stunden) gewährleistet, sondern zugleich bei der ersten Einnahme des Arzneimittels ein rasches Anfluten des Wirkstoffs im Plasma ermöglicht, was zu einer raschen Schmerzlinderung beim Patienten führt ("rapid onset"). Damit kann bei Verabreichung der erfindungsgemäßen Formulierung an einen Schmerzpatienten dessen Schmerz rasch gelindert werden, ohne daß die analgetische Wirkung ebenso rasch wieder nachlassen würde. Die erfindungsgemäße Formulierung vereinigt damit Eigenschaften einer Formulierung mit sofortiger Wirkstofffreisetzung - schnelle Linderung des Schmerzes durch ausreichend hohe Wirkstoffkonzentration kurz nach Gabe des Arzneimittels - mit Eigenschaften einer Formulierung mit verzögerter Freisetzung - langandauemde analgetische Wirkung aufgrund eines ausreichend hohen Wirkstoffspiegels über längere Zeit. Der Schmerzpatient kann somit durch Einnahme des Analgetikums in der erfindungsgemäßen Formulierung seine Schmerzen wirksam akut bekämpfen und zugleich ohne weitere Maßnahmen und lediglich durch regelmäßige Einnahme im Abstand von 12 (oder 24) Stunden effektiv über einen längeren Zeitraum therapieren.

Der Wirkstoff der erfindungsgemäßen Formulierung ist in einer Matrix mit verzögerter Freisetzung enthalten. Es ist jedoch auch denkbar, daß der Wirkstoff in einer Matrix mit üblichem Freisetzungsverhalten enthalten ist und die verzögerte Freisetzung durch eine Überzugsretardierung erreicht wird.

Bei einer weiteren Möglichkeit wird das verzögerte Freisetzungsverhalten durch ein osmotisch getriebenes Freisetzungssystem erreicht.

Für den Fall, daß die erfindungsgemäße Formulierung eine Matrix mit verzögerter Freisetzung enthält, weist die Matrix 1-80 Gew.-% eines oder mehrerer hydrophiler oder hydrophober Polymere als pharmazeutisch annehmbare Matrixbildner auf, beispielsweise Gummis, Celluloseether, Celluloseester, Acrylharze, von Proteinen abgeleitete Materialien, Fette, Wachse, Fettalkohole oder Fettsäureester. Bei der Verwendung hydrophiler Polymere als Matrixbildner ist es bevorzugt, daß die Matrix 5 bis 80 Gew.-% Matrixbildner aufweist.

Ein weiterer Gegenstand der vorliegenden Erfindung ist eine pharmazeutische Formulierung, die 3-(3-Dimethylamino-1-ethyl-2-methylpropyl)phenol oder ein pharmazeutisch annehmbares Salz davon in einer Matrix mit verzögerter Wirkstofffreisetzung enthält, wobei die Matrix 1 bis 80 Gew.-%, insbesondere 5 bis 80 Gew.-%, eines oder mehrerer hydrophiler oder hydrophober Polymeren als pharmazeutisch annehmbaren Matrixbildner enthält und die dadurch gekennzeichnet ist, daß sie als pharmazeutisch annehmbaren Matrixbildner Celluloseether und/oder Celluloseester aufweist, der/die in einer 2 gew.-% wäßrigen Lösung bei 20 °C eine Viskosität von 3.000 bis 150.000 mPa·s aufweist/aufweisen. (Die Viskositätsbestimmung erfolgt dabei mittels Kapillar-Viskosimetrie nach Pharm. Eu.). Die Zusammensetzungen weisen das oben angegebene erfindungsgemäße Freisetzungsprofil auf.

Vorzugsweise werden als pharmazeutisch akzeptable Matrixbildner Celluloseether und/oder Celluloseester eingesetzt, die in einer 2 gew.-%igen wäßrigen Lösung bei 20° C eine Viskosität zwischen 10.000, insbesondere 50.000 mPa·s, und 150.000 mPa·s besitzen.

Besonders geeignete pharmazeutisch akzeptable Matrixbildner sind ausgewählt aus der Gruppe der Hydroxypropylmethylcellulosen (HPMC), Hydroxyethylcellulosen, Hydroxypropylcellulosen (HPC), Methylcellulosen, Ethylcellulosen und Carboxymethylcellulosen und sind insbesondere ausgewählt aus der Gruppe der HPMCs, Hydroxyethylcellulosen und HPCs. Am meisten bevorzugt sind HPMCs mit einer Viskosität von ca. 100.000 mPa·s, gemessen in einer 2 gew.-%igen wäßrigen Lösung bei 20° C.

Der Wirkstoff 3-(3-Dimethylamino-1-ethyl-2-methyl-propyl)phenol kann als solches, d.h. als freie Base, aber auch in Form eines pharmazeutisch akzeptablen Salzes, beispielsweise als Hydrochlorid, vorliegen. Die Herstellung der freien Base ist aus der EP 0 693 475 A1 bekannt. Soweit in der EP 0 693 475 A1 nicht auch die Herstellung pharmazeutisch annehmbarer Salze - wie des Hydrochlorids - offenbart ist, sind diese mittels im Stand der Technik allgemein bekannter Verfahren ausgehend von der freien Base erhältlich.

3-(3-Dimethylamino-1-ethyl-2-methyl-propyl)phenol weist zwei Asymmetriezentren auf, so daß die Verbindung in Form von vier verschiedenen Stereoisomeren vorliegen kann. In der erfindungsgemäßen Formulierung kann 3-(3-Dimethylamino-1-ethyl-2-methyl-propyl)phenol als Gemisch aller vier Diastereomeren in beliebigem Mischungsverhältnis, aber auch als Gemisch von zwei oder drei der vier Stereoisomeren oder in stereoisomerenreiner Form vorliegen. Bevorzugte Stereoisomeren sind hierbei (+)-(1S,2S)-3-(3-Dimethylamino-1-ethyl-2-methyl-propyl)phenol und (-)-(1R,2R)-3-(3-Dimethylamino-1-ethyl-2-methyl-propyl)phenol, die in der erfindungsgemäßen Formulierung als Gemisch, insbesondere als 1:1-Gemisch (Racemat), oder besonders bevorzugt in isomerenreiner Form vorliegen können. Unter "Wirkstoff" ist daher für die Zwecke der vorliegenden Erfindung 3-(3-Dimethylamino-1-ethyl-2-methyl-propyl)phenol als Mischung verschiedener seiner Stereoisomeren oder als eines seiner reinen Stereoisomeren, jeweils als freie Base oder in Form eines pharmazeutisch annehmbaren Salzes, zu verstehen.

In den erfindungsgemäßen Arzneimitteln liegt der verzögert freizusetzende Wirkstoffgehalt vorzugsweise zwischen 0,5 und 85 Gew.-% und der Gehalt an pharmazeutisch akzeptablem Matrixbildner zwischen 8 und 40 Gew.-%. Besonders bevorzugt sind Arzneimittel mit einem verzögert freizusetzenden Wirkstoffgehalt zwischen 3 und 70 Gew.-%, insbesondere zwischen 8 und 66 Gew.-%, und einem Gehalt an pharmazeutisch akzeptablem Matrixbildner zwischen 10 und 35 Gew.-%, insbesondere zwischen 10 und 30 Gew.-%. Wird als Wirkstoff das enantiomerenreine (+)-(1 S,2S)-3-(3-Dimethylamino-1-ethyl-2-methyl-propyl)phenol (oder ein Gemisch der (+)- und (-)-Enantiomeren mit großem Überschuß des (+)-Enantiomeren) verwendet, ist es besonders bevorzugt, daß der Wirkstoffgehalt an der unteren Grenze liegt, d.h. zwischen 0,5 und 25 Gew.-% (bezogen auf das Gesamtgewicht). Wird als Wirkstoff das enantiomerenreine (-)-(1R,2R)-3-(3-Dimethylarnino-1-ethyl-2-methyl-propyl)phenol (oder ein Gemisch der (+)- und (-)-Enantiomeren mit großem Überschuß des (-)-Enantiomeren) verwendet, ist es besonders bevorzugt, daß der Wirkstoffgehalt zwischen 16 und 66 Gew.-% liegt.

Weitere Bestandteile der Matrix der erfindungsgemäßen Formulierung können ggf. verdauliche langkettige (d.h. mit 8 bis 50 C-Atomen, bevorzugt 12 bis 40 C-Atomen) unsubstiuierte oder substituierte Kohlenwasserstoffe, wie z.B. Fettalkohole, Fettsäurenglycerylester, Mineral- und Pflanzenöle sowie Wachse sein, wobei Kohlenwasserstoffe mit einem Schmelzpunkt zwischen 25° und 90° C bevorzugt sind. Insbesondere sind Fettalkohole bevorzugt, ganz besonders Laurylalkohol, Myristylalkohol, Stearylalkohol, Cetylalkohol und Cetylstearylalkohol. Ihr Gehalt in der Matrix beträgt 0 bis 60 Gew.-%. Alternativ oder zusätzlich können auch Polyethylenglycole mit einem Gehalt von 0 bis 60 Gew.-% in der Matrix enthalten sein.

In den erfindungsgemäßen pharmazeutischen Formulierungen können ferner als weitere Bestandteile pharmazeutisch gebräuchliche Hilfsstoffe wie Füllstoffe, beispielsweise Lactose, mikrokristalline Cellulose (MCC) oder Calciumhydrogenphosphat, sowie Gleit-, Schmier- und Fließregulierungsmittel, beispielsweise Talkum, Magnesiumstearat, Stearinsäure und/oder hochdisperses Siliciumdioxid, enthalten sein, deren Gesamtgehalt in der Tablette zwischen 0 und 80 Gew.-%, vorzugsweise zwischen 5 und 65 Gew.-% liegt.

Vielfach ist die Freisetzungsgeschwindigkeit eines Wirkstoffes aus einer Darreichungsform vom pH-Wert des Freisetzungsmediums abhängig. Dieser kann während der Gastrointestinalpassage des Arzneimittels in einem pH-Wert-Bereich von unter 1 bis etwa 8 schwanken. Diese Schwankungen können von einer einnehmenden Person zur anderen verschieden sein. Auch kann bei ein und derselben Person von einer Einnahme zur anderen ein unterschiedliches pH-Wert-Zeit-Profil während der Gastrointestinalpassage gegeben sein. Ist die Freisetzungsgeschwindigkeit des Wirkstoffes aus dem Arzneimittel vom pH-Wert abhängig, so kann dies zu unterschiedlichen Freisetzungsgeschwindigkeiten in vivo und damit unterschiedlicher Bioverfügbarkeit führen. Die Freisetzungsprofile des Wirkstoffs (in Form der Base oder eines seiner pharmazeutisch akzeptablen Salze) aus einer erfindungsgemäßen pharmazeutischen Formulierung sind jedoch überraschenderweise unabhängig vom pH-Wert, wie er physiologisch während der Gastrointestinalpassage auftreten kann. Die Freisetzungsprofile bei einem Umgebungs-pH-Wert von 1,2, 4,0 und 6,8 sind sowohl untereinander identisch als auch im Vergleich zur Freisetzung während eines pH-Wert-Zeit-Profils von pH 1,2 über pH 2,3 und pH 6,8 bis zu pH 7,2.

Es hat sich gezeigt, daß es für die Erreichung der verzögerten Wirkstofffreisetzung aus der bevorzugt in Tablettenform vorliegenden erfindungsgemäßen Formulierung unerheblich ist, ob bei ansonsten unveränderten Abmessungen und unveränderter Zusammensetzung der Tablette, bezogen auf den Wirkstoff, den Matrixbildner und die fakultativen Bestandteile, als Füllstoff ein wasserlöslicher Füllstoff, beispielsweise Lactose, ein unlöslicher, in wäßrigem Medium nicht quellender Füllstoff, beispielsweise Calciumhydrogenphosphat, oder ein unlöslicher, in wäßrigem Medium quellender Füllstoff, beispielsweise mikrokristalline Cellulose, eingesetzt wird. Alle derartigen Arzneimittel zeigen ein einander entsprechendes Freisetzungsverhalten.

Überraschend ist ferner, daß in den erfindungsgemäßen Zusammensetzungen bei gegebener Wirkstoffmenge die Menge an Matrixbildner und die Menge der fakultativen Bestandteile jeweils über einen relativ großen Bereich variieren können, ohne daß die therapeutische Wirksamkeit von mindestens 12 h bzw. bei zweimal täglicher Applikation in Frage gestellt werden würde (solange die oben angegebenen Mengengrenzen für Wirkstoff, Matrixbildner und die weiteren, fakultativen Bestandteile eingehalten werden). Eine Wirksamkeit über mindestens 12 h ist z.B. bei einem Wirkstoff-Gehalt von ca. 32,25 Gew.-% (bezogen auf das Gewicht der Gesamtzusammensetzung) sowohl in einer Zusammensetzung aus ca. 12,9 Gew.-% HPMC mit einer Viskosität von 100.000 mPa·s als Matrixbildner und einem Gehalt an beispielsweise MCC als Füllstoff von ca. 52,6 Gew.-% als auch in einer Zusammensetzung aus ca. 25,8 Gew.-% derselben HPMC und ca. 39,7 Gew.-% MCC (oder Lactose-Monohydrat) bei sonst gleichen Mengen an Gleit-, Schmier- und Fließregulationsmitteln gewährleistet. Vergleichbares gilt für erfindungsgemäße Zusammensetzungen mit einem höheren oder geringeren Wirkstoff-Gehalt innerhalb der angegebenen Grenzen.

Außerordentlich überraschend ist auch die Beobachtung, daß bei Verabreichung der erfindungsgemäßen pharmazeutischen Formulierungen mit verzögerter Wirkstofffreisetzung bei menschlichen Versuchspersonen trotz des hohen First-pass-Effekts für den Wirkstoff wider Erwarten eine im Vergleich zu Formulierungen mit sofortiger Wirkstofffreisetzung unveränderte Bioverfügbarkeit erreicht wird.

Es sind ferner solche erfindungsgemäßen Zusammensetzungen bevorzugt, deren tₘₐₓ-Wert im Plasmakonzentrations-Zeit-Diagramm in vivo zwischen 2 und 10 h, insbesondere zwischen 3,5 und 6 h und ganz besonders bevorzugt zwischen 4 und 5,5 h nach oraler Verabreichung der Zusammensetzung beträgt, d.h. deren Peak-Plasma-Level in den genannten Zeiträumen auftritt.

Die erfindungsgemäße Formulierung enthält den Wirkstoff 3-(3-Dimethylamino-1-ethyl-2-methyl-propyl)phenol als solches und/oder als pharmazeutisch annehmbares Salz in einer Menge von üblicherweise 2,5 bis 800 mg, insbesondere 5 bis 400 mg, ganz besonders bevorzugt 10 bis 250 mg (Gewicht des Wirkstoffs 3-(3-Dimethylamino-1-ethyl-2-methylpropyl)phenol als Hydrochlorid) pro Dosierungseinheit, wobei das Freisetzungsverhalten der erfindungsgemäßen Formulierung durch die exakte Menge des Wirkstoffs nicht beeinflußt wird, solange die oben angegebenen Gehaltsgrenzen eingehalten werden. Aufgrund der unterschiedlichen Wirkstärke der beiden besonders bevorzugten Enantiomeren (+)-(1S,2S)-3-(3-Dimethylamino-1-ethyl-2-methylpropyl)phenol und (-)-(1R,2R)-3-(3-Dimethylamino-1-ethyl-2-methylpropyl)phenol ist es bevorzugt, daß das wirkstärkere (+)-(1S,2S)-3-(3-Dimethylamino-1-ethyl-2-methyl-propyl)phenol in einer Menge von 2,5 bis 80 mg, insbesondere 5 bis 40 mg und ganz besonders bevorzugt in einer Menge von 10 bis 25 mg Wirkstoff (bezogen auf das Hydrochlorid) in den erfindungsgemäßen Formulierungen vorliegt, während das (-)-(1R,2R)-3-(3-Dimethylamino-1-ethyl-2-methyl-propyl)phenol vorzugsweise in einer Menge von 25 bis 800 mg, insbesondere 50 bis 400 mg und ganz besonders bevorzugt in einer Menge von 100 bis 250 mg Wirkstoff (bezogen auf das Hydrochlorid) in den erfindungsgemäßen Formulierungen vorliegt, und zwar unter der Maßgabe, daß die oben angegebenen Gehaltsgrenzen eingehalten werden.

Pharmazeutisch annehmbare (oder akzeptable) Salze des Wirkstoffs sind im Sinne dieser Erfindung solche Salze des Wirkstoffs, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Die erfindungsgemäßen pharmazeutischen Formulierungen können sowohl als einfache Tablette als auch als überzogene Tablette, beispielsweise als Filmtablette oder Dragee vorliegen. Üblicherweise sind die Tabletten rund und bikonvex; möglich sind auch oblonge Tablettenformen, die eine Teilbarkeit der Tablette gestatten. Ferner sind auch Granulate, Spheroide, Pellets oder Mikrokapseln möglich, die in Sachets oder Kapseln gefüllt werden oder zu zerfallenden Tabletten verpreßt werden können.

Für die überzogenen Tabletten können ein oder mehrere Überzugsschichten verwendet werden. Als Überzugsmaterial eignen sich bekannte Hydroxypropylmethylcellulosen mit niedriger Viskosität von ca. 1 bis 100 mPa·s und niedrigem Molekulargewicht von < 10.000 (z.B. Pharmacoat 606 mit einer Viskosität von 6 mPa·s in einer 2 gew.-%igen wäßr. Lösung bei 20 °C), die das Freisetzungsprofil der erfindungsgemäßen Arzneimittel nur geringfügig beeinflussen. Dem Fachmann bekannte Diffusionsüberzüge, beispielsweise auf Basis von quellbaren, aber wasserunlöslichen Poly(meth)acrylaten, führen zu einer Modulation der Verzögerung der Wirkstofffreisetzungen aus erfindungsgemäßen pharmazeutischen Formulierungen. Der wirkstoffhaltige, den Wirkstoff retardiert freisetzende Tablettenkem mit einem Wirkstoffgehalt vorzugsweise zwischen 0,5 und 85 Gew.-%, besonders bevorzugt zwischen 3 und 70 Gew.-% und ganz besonders bevorzugt zwischen 8 und 66 Gew.-%, kann mit zusätzlichem Wirkstoff, der nicht retardiert als Initialdosis freigesetzt wird, durch verschiedene, dem Fachmann bekannte Verfahren, beispielsweise Dragieren, Aufsprühen aus Lösungen oder Suspensionen oder durch Pulverauftragverfahren, umhüllt sein, ohne daß das für die gewünschte verzögerte Freisetzung bei gleichzeitigem raschen Anfluten des Wirkstoffs zur schnellen Schmerzlinderung bei erster Gabe der erfindungsgemäßen pharmazeutischen Formulierung zwingend erforderlich ist. Weitere Ausführungsformen stellen Mehrschicht- und Manteltabletten dar, bei denen 3-(3-Dimethylamino-1-ethyl-2-methyl-propyl)phenot oder eines seiner pharmzeutisch akzeptablen Salze in einer oder mehreren Schichten der Mehrschichttablette mit einem Wirkstoffgehalt vorzugsweise zwischen 0,5 und 85 Gew.-%, besonders bevorzugt zwischen 3 und 70 Gew.% und ganz besonders bevorzugt zwischen 8 und 66 Gew.-% bzw. im Kern der Manteltablette mit einem Wirkstoffgehalt vorzugsweise zwischen 0,5 und 85 Gew.-%, besonders bevorzugt zwischen 3 und 70 Gew.% und ganz besonders bevorzugt zwischen 8 und 66 Gew.% durch einen pharmazeutisch akzeptablen Matrixbildner retardiert freigesetzt wird und die Freisetzung des Wirkstoffs in einer oder mehreren Schichten der Mehrschichttablette bzw. der äußeren Mantelschicht der Manteltabletten unretardiert erfolgt. Mehrschicht- und Manteltabletten können ein oder mehrere wirkstofffreie Überzüge enthalten.

An Stelle einer verzögert freisetzenden Matrix in der pharmazeutischen Formulierung mit verzögerter Freisetzung ist auch die Verwendung einer normal freisetzenden Matrix mit einem die Freisetzung des Wirkstoffs retardierenden Überzug möglich. Dabei kann z.B. der Wirkstoff in einer üblichen Matrix aus mikrokristalliner Cellulose und ggf. weiteren pharmazeutischen Hilfsstoffen, wie etwa Bindemittel, Füllstoffe, Gleit-, Schmier- und Fließregulierungsmittel, enthalten sein, die mit einem Material überzogen bzw. beschichtet werden, welche die verzögerte Freisetzung des Wirkstoffs in wäßrigem Medium steuern. Geeignete Beschichtungsmittel sind z.B. wasserunlösliche Wachse und Polymere, wie Polymethacrylate (Eudragit o.ä.) oder wasserunlösliche Cellulosen, insbesondere Ethylcellulose. Ggf. können im Überzugsmaterial auch wasserlösliche Polymere, wie Polyvinylpyrrolidon, wasserlösliche Cellulosen, wie Hydroxypropylmethylcellulose oder Hydroxypropylcellulose, andere wasserlösliche Mittel, wie Polysorbat 80, oder hydrophile Porenbildner, wie Polyethylenglycol, Lactose oder Mannitol, enthalten sein.

Neben oder in Ergänzung zu den Möglichkeiten einer verzögert freisetzenden Matrix in der pharmazeutischen Formulierung mit verzögerter Freisetzung oder einer normal freisetzenden Matrix mit einem die Freisetzung des Wirkstoffs retardierenden Überzug kann auch ein osmotisch getriebenes Freisetzungssystem zum Erreichen einer verzögerten Freisetzung verwendet werden. Bei einem solchen, bevorzugt oralen, Freisetzungssystem ist mindestens eine, bevorzugt sämtliche, Oberfläche/n des Freisetzungsystems, bevorzugt die, die mit dem Freisetzungsmedium in Kontakt steht/stehen oder stehen könnte/können, semipermeabel, vorzugsweise mit einem semipermeablen Überzug ausgestattet, so dass die Oberfläche/n für das Freisetzungsmedium durchlässig, für den Wirkstoff aber im wesentlichen, bevorzugt vollständig, undurchlässig ist/sind, wobei die Oberfiäche/n und/oder gegebenenfalls der Überzug wenigstens eine Öffnung zur Freisetzung des Wirkstoffes aufweist/aufweisen. Der Wirkstoff 3-(3-Dimethylamino-1-ethyl-2-methylpropyl)phenol oder ein pharmazeutisch annehmbares Salz davon, vorzugsweise (+)-(1S,2S)-3-(3-Dimethylamino-1-ethyl-2-methylpropyl)phenol oder ein pharmazeutisch annehmbares Salz davon und/oder (-)-(1R,2R)-3-(3-Dimethylamino-1-ethyl-2-methyl-propyl)phenol oder ein pharmazeutisch annehmbares Salz davon, bzw. deren Mischung, kann dabei - muss aber nicht - in einer Matrix vorliegen. Vorzugsweise ist darunter ein System in Tablettenform mit einer Abgabeöffnung, einem osmotischen Arzneimittelkern, einer semipermeablen Membran und einem polymeren Teil, der Druck ausübt, zu verstehen. Ein gutes und bevorzugtes Beispiel für ein solches System ist dabei das OROS®-System der ALZA Corporation, USA, deren Internetauftritt oder andere Produktinformationen Einzelheiten über das OROS®-System enthält. Insbesondere sind dies auch das OROS® Push-Pull™-System, das OROS® Delayed Push-Pull™-System, das OROS® Multi-Layer Push-Pull™-system, das OROS® Push-Stick System, bzw. auch in bestimmten Fragestellungen das L-OROS™. Ausführungsformen und Beispiele der konkreten Herstellung osmotisch getriebener Freisetzungssystem sind den US Patenten US 4,765,989, US 4,783,337 und US 4,612,008 zu entnehmen, die vollinhaltlich Bestandteil der Beschreibung dieser Erfindung sind.

Die erfindungsgemäßen Zusammensetzungen können beispielsweise nach folgendem allgemeinen Verfahren hergestellt werden:
Die Bestandteile der Zusammensetzung (Wirkstoff, Matrixbildner und fakultative Bestandteile) werden der Reihe nach eingewogen und anschließend auf einer üblichen Siebmaschine gesiebt. Hier kann beispielsweise die Siebmaschine Quadro Comil U10 eingesetzt werden, wobei eine gebräuchliche Siebgröße ca. 0,813 mm beträgt. Die Siebung wird anschließend in einem Containermischer gemischt, z.B. in einem Bohle Containermischer; typische Arbeitsbedingungen sind: Dauer ca. 15 min ± 45 s bei einer Drehzahl von 20 ± 1 U/min. Danach wird die erhaltene Pulvermischung auf einer Tablettenpresse zu einer Tablette verpreßt. Hierfür kann z.B eine Tablettenpresse Korsch EKO mit einem drageegewölbten Rundstempel mit 10 mm Durchmesser Verwendung finden. Alternativ kann auch eine Kompaktierung der Pulvermischung und anschließende Siebung (Comill 3 mm Reibschnitzelsieb und anschließend 1,2 mm Rundlochsieb) der Preßlinge erfolgen, wobei das so entstehende Granulat anschließend wie oben beschrieben unter Zusatz von Schmiermittel (z.B. Magnesiumstearat) auf z.B. einer EK0 Tablettenpresse mit 10 mm Rundstempeln verpreßt wird. Die Granulation kann auch durch Naßgranulation auf Basis wäßriger oder organischer Lösungsmittel erfolgen; bevorzugt sind dabei wäßrige Lösungsmittel mit oder ohne geeignete Bindemittel. Das Herstellungsverfahren kann ohne weiteres an die jeweiligen Erfordernisse und die gewünschte Darreichungform nach im Stand der Technik wohlbekannten Vorgehensweisen angepaßt werden.

Die Herstellung erfindungsgemäßer pharmazeutischer Formulierungen ist durch eine hohe Reproduzierbarkeit der Freisetzungseigenschaften der erhaltenen Zusammensetzungen, die 3-(3-Dimethylamino-1-ethyl-2-methylpropyl)phenol oder eines seiner pharmzeutisch akzeptablen Salze enthalten, gekennzeichnet. Während einer Lagerzeit von mindestens einem Jahr unter den üblichen Lagerungsbedingungen gemäß ICH Q1AR-Stability-Testing-Guideline erweist sich das Freisetzungsprofil erfindungsgemäßer Arzneimittel als stabil.

Bei täglich ein- oder zweimaliger Einnahme einer erfindungsgemäßen pharmazeutischen Formulierung durch den Patienten wird eine gute therapeutische Wirksamkeit bei anhaltend starken Schmerzen sicher erzielt.

### Beispiele

Die Beispiele dienen der Illustration der vorliegenden Erfindung und bevorzugter Ausführungsformen, sollen aber ihren Schutzumfang nicht beschränken.

### Beispiel 1

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Fa. Shinetsu), 100.000 mPa·s | 80 mg |
| Mikrokristalline Cellulose (Avicel PH 102 von Fa. FA. FMC) | 123 mg |
| Hochdisperses Siliciumdioxid | 4 mg |
| Magnesiumstearat | 3 mg |
| Gesamtmenge | 310 mg |

wurden in einer Ansatzgröße von 1000 Tabletten in folgender Weise hergestellt:
Alle Bestandteile wurden eingewogen und auf einer Siebmaschine Quadro Comil U10 unter Verwendung einer Siebgröße von 0,813 mm gesiebt, in einem Contäinermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Die Freisetzung in vitro wurde bestimmt unter Anwendung der Ph. Eur. Paddle Method bei 75 U/min in 900 ml Puffer pH 6,8 nach Ph. Eur. bei 37 °C und mit UV-spektrometrischem Nachweis und ist in folgender Tabelle wiedergegeben.

| **Zeit [min]** | **Freigesetzte Gesamtmenge des Wirkstoffs [%]** |
|---|---|
| 0 | 0 |
| 30 | 18 |
| 60 | 27 |
| 120 | 41 |
| 180 | 50 |
| 240 | 59 |
| 360 | 71 |
| 480 | 80 |
| 600 | 87 |
| 720 | 93 |

### Beispiel 2

### 3000 Matrixtabletten mit folgender Zusammensetzung pro Tablette

| | |
|---|---|
| (-)-(1R,2R)-3-(3-Dimethy)amino-1-ethyl-2-methyl-propyl)phenol-Hydrochlorid | 200 mg |
| Hydroxypropylmethylcellulose (Metolose 90 SH 100.000 von Fa. Fa. Shinetsu), 100.000 mPa·s | 80 mg |
| Mikrokristalline Cellulose (Avicel PH 102 von Fa. FA. FMC) | 23 mg |
| Hochdisperses Siliciumdioxid | 4 mg |
| Magnesiumstearat | 3 mg |
| Gesamtmenge | 310 mg |

wurden analog zu dem in Beispiel 1 angebenen Verfahren hergestellt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 19 |
| 60 | 30 |
| 120 | 46 |
| 180 | 58 |
| 240 | 68 |
| 360 | 84 |
| 480 | 93 |
| 720 | 99 |

### Beispiel 3

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 40 mg |
| Mikrokristalline Cellulose (Avicel PH 102 von Fa. FMC) | 163 mg |
| Hochdisperses Siliciumdioxid | 4 mg |
| Magnesiumstearat | 3 mg |
| Gesamtmenge | 310 mg |

wurden in einer Ansatzgröße von 3000 Tabletten analog zu dem in Beispiel 1 angebenen Verfahren hergestellt.
Die Freisetzung in vitro wurde wie in Beispiel 1 bestimmt; zusätzlich wurde unter sonst gleichen Bedingungen die Freisetzung bei einer Rührgeschwindigkeit von 50 U/min und 100 U/min ermittelt.

| **Zeit [min]** | **Freigesetzte Gesamtmenge des Wirkstoffs [%] bei 50 U/min** | **Freigesetzte Gesamtmenge des Wirkstoffs [%] bei 75 U/min** | **Freigesetzte Gesamtmenge des Wirkstoffs [%] bei 100 U/min** |
|---|---|---|---|
| 0 | 0 | 0 | 0 |
| 30 | 20 | 20 | 21 |
| 60 | 35 | 33 | 35 |
| 120 | 54 | 51 | 53 |
| 180 | 67 | 63 | 66 |
| 240 | 76 | 73 | 76 |
| 360 | 89 | 87 | 89 |
| 480 | 97 | 95 | 97 |
| 600 | 100 | 100 | 100 |

### Beispiel 4

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 80 mg |
| Lactose-Monohydrat 230 (Fa. Meggle) | 123 mg |
| Hochdisperses Siliciumdioxid | 4 mg |
| Magnesiumstearat | 3 mg |
| Gesamtmenge | 310 mg |

wurden in einer Ansatzgröße von 200 Tabletten analog zu dem in Beispiel 1 angebenen Verfahren hergestellt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 16 |
| 60 | 26 |
| 120 | 39 |
| 180 | 49 |
| 240 | 57 |
| 360 | 71 |
| 480 | 81 |
| 600 | 87 |
| 720 | 92 |

### Beispiel 5

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 40 mg |
| Cellactose 80 (Fa. Meggle) | 163 mg |
| Hochdisperses Siliciumdioxid | 4 mg |
| Magnesiumstearat | 3 mg |
| Gesamtmenge | 310 mg |

wurden in einer Ansatzgröße von 100 Tabletten analog zu dem in Beispiel 1 angebenen Verfahren hergestellt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 18 |
| 60 | 31 |
| 120 | 48 |
| 180 | 61 |
| 240 | 71 |
| 360 | 84 |
| 480 | 91 |
| 600 | 95 |
| 720 | 97 |

### Beispiel 6

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 80 mg |
| Ludipress (Fa. BASF) | 123 mg |
| Hochdisperses Siliciumdioxid | 4 mg |
| Magnesiumstearat | 3 mg |
| Gesamtmenge | 310 mg |

wurden in einer Ansatzgröße von 100 Tabletten analog zu dem in Beispiel 1 angebenen Verfahren hergestellt.
Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 17 |
| 60 | 27 |
| 120 | 40 |
| 180 | 51 |
| 240 | 59 |
| 360 | 72 |
| 480 | 82 |
| 600 | 89 |
| 720 | 93 |

### Beispiel 7

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 50 mg |
| Hydroxypropylmethylcellulose (Metolose 90 SH 100.000 von Fa. Shinetsu), 100.000 mPa·s | 40 mg |
| Mikrokristalline Cellulose (Avicel PH 102, Fa. FMC) | 163 mg |
| Lactose 200 (Fa. Meggle) | 50 mg |
| Hochdisperses Siliciumdioxid | 4 mg |
| Magnesiumstearat | 3 mg |
| Gesamtmenge | 310 mg |

wurden in einer Ansatzgröße von 200 Tabletten analog zu dem in Beispiel 1 angebenen Verfahren hergestellt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 18 |
| 60 | 31 |
| 120 | 49 |
| 180 | 61 |
| 240 | 70 |
| 360 | 82 |
| 480 | 90 |
| 600 | 94 |
| 720 | 96 |

### Beispiel 8

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Cellactose (Fa. Meggle) | 72,5 mg |
| Hydroxyethylcellulose (Natrosol 250 HX von Fa. Herkules) | 12,5 mg |
| Cutina HR (Fa. Henkel) | 130 mg |
| Talkum | 3 mg |
| Magnesiumstearat | 2 mg |
| Gesamtmenge | 320 mg |

wurden in einer Ansatzgröße von 200 Tabletten wie folgt hergestellt:
Der Wirkstoff, Cellactose, Natrosol und Cutina wurden gemischt, dann im Trockenschrank auf 80 °C erhitzt und in einem Kenwood Chef-Küchenmischer granuliert. Das abgekühlte Granulat wurde durch ein 1 mm-Sieb gesiebt. Nach Abmischen mit Magnesiumstearat und Talkum wurde das Granulat auf einer EK 0 Exzeterpresse (Korsch) zu 6 x 15 mm großen Oblong-Tabletten mit Bruckkerbe verpreßt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 28 |
| 60 | 39 |
| 120 | 56 |
| 180 | 68 |
| 240 | 80 |
| 360 | 97 |
| 390 | 99 |

### Beispiel 9

### Matrixtabletten mit folgender Zusammensetzung pro Tablette

| | |
|---|---|
| (+)-(1S,2S)-3-(3-Dimethylamino-1-ethyl-2-methyl-propyl)phenol-Hydrochlorid | 10 mg |
| Hydroxypropylmethylcellulose (Metolose 90 SH 100.000 von Fa. Shinetsu), 100.000 mPa·s | 80 mg |
| Mikrokristalline Cellulose (Avicel PH 102, Fa. FMC) | 213 mg |
| Lactose 200 (Fa. Meggle) | 50 mg |
| Hochdisperses Siliciumdioxid | 4 mg |
| Magnesiumstearat | 3 mg |
| Gesamtmenge | 310 mg |

wurden in einer Ansatzgröße von 100 Tabletten analog zu dem in Beispiel 1 angebenen Verfahren hergestellt.

Die Freisetzung in vitro wurde wie in Beispiel 1 bestimmt.

| **Zeit [min]** | **Freigesetzte Gesamtmenge des Wirkstoffs [%]** |
|---|---|
| 0 | 0 |
| 30 | 15 |
| 60 | 24 |
| 120 | 36 |
| 180 | 44 |
| 240 | 51 |
| 360 | 61 |
| 480 | 69 |
| 600 | 75 |
| 720 | 79 |

### Beispiel 10

### Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 80 mg |
| Mikrokristalline Cellulose (Avicel PH 102 von Fa. FMC) | 63 mg |
| Hochdisperses Siliciumdioxid | 4 mg |
| Magnesiumstearat | 3 mg |
| Gesamtmenge | 250 mg |

wurden in einer Ansatzgröße von 100 Tabletten analog zu dem in Beispiel 1 angebenen Verfahren hergestellt.

Die Freisetzung in vitro wurde unter folgenden Bedingungen bestimmt:
(A) Anwendung der Ph. Eur. Paddle Method bei 75 U/min in 900 ml Puffer pH 7,2 nach USP 22 bei 37 °C und mit UV-spektrometrischem Nachweis
(B) Anwendung der Ph. Eur. Paddle Method bei 75 U/min, wobei von 0-30 min ein pH von 1,2, von 30-120 min ein pH von 2,3, von 120-180 min ein pH von 6,5 und für die restliche Versuchszeit ein pH von 7,2 eingestellt wurde. Die Tabelle gibt die Ergebnisse für beide Versuchsbedingungen wieder:

| **Zeit [min]** | **Freigesetzte Gesamtmenge des Wirkstoffs [%] unter Bedingung (A)** | **Freigesetzte Gesamtmenge des Wirkstoffs [%] unter Bedingung (B)** |
|---|---|---|
| 0 | 0 | 0 |
| 30 | 19 | 20 |
| 60 | 29 | 30 |
| 120 | 43 | 44 |
| 180 | 54 | 55 |
| 240 | 63 | 65 |
| 360 | 78 | 80 |
| 480 | 87 | 90 |
| 600 | 94 | 97 |
| 720 | 98 | 100 |

Der Versuch zeigt, daß das Freisetzungsverhalten der erfindungsgemäßen Formulierungen unabhängig vom pH-Wert des Freisetzungsmediums ist.

### Beispiel 11

### Pellets folgender Zusammensetzung

| | |
|---|---|
| (-)-(1R,2R)-3-(3-Dimethylamino-1-ethyl-2-methyl-propyl)phenol-Hydrochlorid | 100 mg |
| Niedrigsubstituierte Hydroxypropylcellulose(L-HPC LH 31 von Fa. Shinetsu | 75 mg |
| Aquacoat (wäßr. Ethylcellulose-Dispersion; von Fa. FMC) (als Trockensubstanz gerechnet) | 20 mg |
| Mikrokristalline Cellulose (Avicel PH 101, Fa. FMC) | 75 mg |
| Dibutylsebacat (DBS) | 4 mg |
| Tween 80 | 0,4 mg |
| Gesamtmenge | 274,4 mg |

wurden wie folgt hergestellt:
Der Wirkstoff, Avicel und L-HPC wurden in einem Planetenmischer (Kenwood K-Mischer) 10 min gemischt und anschließend mit Wasser granuliert. Das feuchte Granulat wurde im Nica-Extruder mit einer 0,8 x 0,8 mm Matrize extrudiert und dann 10 min im Nica-Spheronizer bei 500 U/min ausgerundet (Beladung 1 kg). Die Pellets wurden über Nacht im Trockenschrank bei 50 °C getrocknet und anschließend in Siebfraktionen klassiert.
Pellets der Größe 0,6-1,0 mm (Ausbeute ca. 95%) wurden in der WSG (Glatt GPCG1 mit Wurstereinsatz) bei Zulufttemperaturen von 60 °C (Produkttemperatur 40 °C) mit einer wäßrigen Dispersion aus Aquacoat und DBS (20 %, berechnet auf Aquacoat Feststoffgehalt) überzogen, so daß sie eine Gewichtszunahme von 9,8 % (bezogen auf das Ausgangsgewicht) aufwiesen. Die Herstellung der Dispersion erfolgte gemäß Herstellerangaben (FA. FMC), wobei das DBS zusammen mit Tween 80 in einer Teilmenge des Wassers homogenisiert und dann zur verdünnten Aquacoat-Dispersion hinzugegeben wurde. Die fertige Dispersion hatte einen Feststoffgehalt von 20 Gew.-% und wurde mind. 3h gerührt. Die überzogenen Pellets wurden in der WSG getrocknet und im Trockenschrank getempert (2h bei 60 °C). Die Prüfung der Freisetzung erfolgte analog Beispiel 1, jedoch nach der Körbchenmethode bei 100 U/min.

| **Zeit [min]** | **Freigesetzte Gesamtmenge des Wirkstoffs [%]** |
|---|---|
| 0 | 0 |
| 30 | 5 |
| 60 | 15 |
| 120 | 28 |
| 180 | 43 |
| 240 | 56 |
| 360 | 73 |
| 480 | 82 |
| 600 | 87 |
| 720 | 90 |

### Klinische Studie

In einer monozentrischen, offenen, randomisierten Einzeldosis-4-Weg-Crossover-Studie wurden zur Ermittlung pharmakokinetischer Daten verschiedene Darreichungsformen von (-)-(1R,2R)-3-(3-Dimethylamino-1-ethyl-2-methyl-propyl)phenol-Hydrochlorid (Wirkstoff) an 16 gesunde männliche weiße Probanden im Alter von 18 bis 45 Jahren verabreicht. Die experimentelle Bestimmung der Daten erfolgte mittels HPLC-Analytik.

### Verabreicht wurden

| | |
|---|---|
| "Capsule 100 mg" | Kapseln mit sofortiger Freisetzung des Wirkstoffs und einer Wirkstoffmenge von 100 mg |
| "Capsule 25 mg" | Kapseln mit sofortiger Freisetzung des Wirkstoffs und einer Wirkstoffmenge von 25 mg |
| "Tablet 100 mg" | Tablette gemäß Beispiel 1 (Wirkstoffmenge 100 mg) |
| "Tablet 200 mg" | Tablette gemäß Beispiel 2 (Wirkstoffmenge 200 mg) |
| | |

(Bei den Kapseln handelte es sich um weiß-opake Hartgelatine-Kapseln der Größe 0 EL mit einer Füllmenge von 360 mg, die sich wie folgt zusammensetzten:
"Capsule 100 mg": 100 mg (-)-(1R,2R)-3-(3-Dimethylamino-1-ethyl-2-methyl-propyl)phenol-Hydrochlorid, 152 mg Mikriokristalline Cellulose, 8 mg Aerosil, 20 mg Magnesiumstearat und 80 mg Primojel (NatriumCarboxymethylstärke Typ A von Fa. Avebe);
"Capsule 25 mg": 25 mg (-)-(1R,2R)-3-(3-Dimethylamino-1-ethyl-2-methylpropyl)phenol-Hydrochlorid, 227 mg Mikriokristalline Cellulose, 8 mg Aerosil, 20 mg Magnesiumstearat und 80 mg Primojel (NatriumCarboxymethylstärke Typ A von Fa. Avebe))

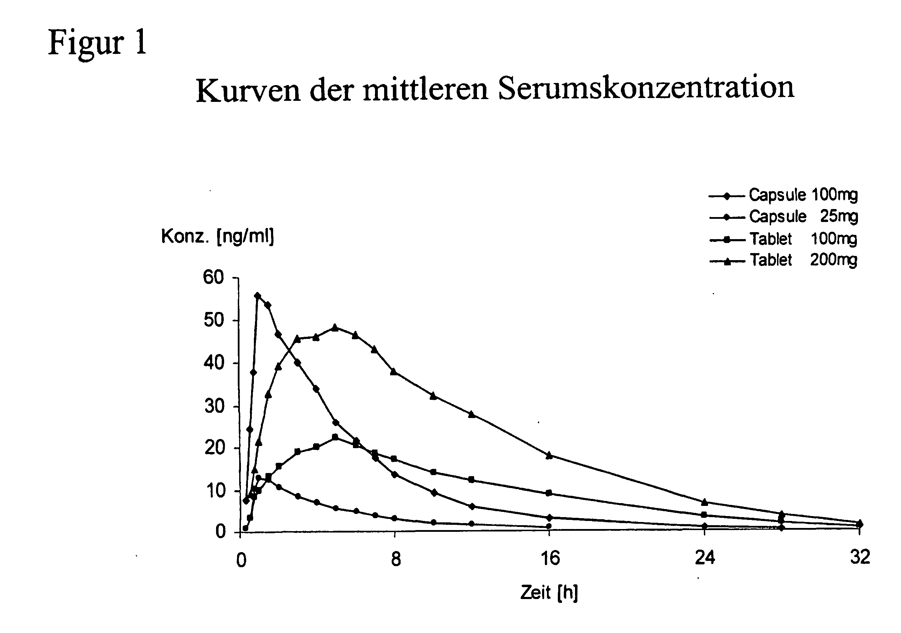

Die wesentlichen pharmakokinetischen Daten sind in der nachstehenden Tabelle, der Verlauf der experimentell ermittelten mittleren Serumskonzentrationen in Figur 1 wiedergegeben.

| **Parameter** | **"Capsule 25 mg"** | **"Capsule 100 mg"** | **"Tablet 100 mg"** | **"Tablet 200 mg"** |
|---|---|---|---|---|
| AUC [ng·h/ml] | 69 ± 14 | 318 ± 66 | 300 ± 51 | 667 ± 141 |
| cₘₐₓ [ng/ml] | 14 ± 4 | 64 ± 19 | 23 ± 5 | 51 ± 13 |
| tₘₐₓ [h] | 1,2 ± 0,4 | 1,5 ± 0,9 | 4,6 ± 1,3 | 4,8 ± 1,1 |
| MRT* [h] | 5,8 ± 0,7 | 5,9 ± 0,9 | 10,7 ± 1,5 | 10,3 ± 1,1 |
| HVD** | 3,5 ± 1,2 | 3,6 ± 1,1 | 12,4 ± 2,8 | 11,9 ± 2,3 |

| | | | | |
|---|---|---|---|---|
| *MRT = "Mean Residence Time" = "Mittlere Aufenthaltszeit im Körper" | | | | |
| **HVD = "Half Value Duration" = "Halbwert-Dauer" | | | | |

Zum einen zeigt sich gerade im Vergleich zwischen "Capsule 100 mg" und "Tablet 100 mg", dass die erfindungsgemäßen Formulierungen die Aufgabe, eine 3-(3-Dimethylamino-1-ethyl-2-methyl-propyl)phenol enthaltende pharmazeutische Formulierung mit verzögerter Wirkstofffreisetzung bereitzustellen, ausgezeichnet erfüllen. Zum anderen zeigt sich auch eine sehr günstige Dosisproportionalität im Freisetzungsverhalten im Vergleich zwischen "Tablet 100 mg" und "Tablet 200 mg".

Weiter läßt sich daraus aber auch ersehen, daß die beiden erfindungsgemäßen Zusammensetzungen "Tablet 100 mg" und "Tablet 200 mg" am Anfang den Wirkstoff in spürbarer Menge, jedoch langsamer freisetzen als die beiden Formulierungen mit sofortiger Freisetzung; die Plasmaspiegel sind jedoch bei beiden retardierten Formulierungen bereits nach 1 h größer als 10 ng/ml und auch nach 16 h noch ausreichend hoch, um eine analgetische Wirkung zu gewährleisten. Simulationsstudien für "Tablet 100 mg" haben darüberhinaus gezeigt, daß bei wiederholter Gabe des Arzneimittels im Abstand von 12 h Serumspiegel erreicht werden, die nicht unter 20 ng/ml fallen, so daß bereits durch 2-mal tägliche Einnahme eine gute analgetische Wirksamkeit gewährleistet ist. Das bedeutet einen großen Fortschritt für die Behandlung insbesondere chronischer Schmerzzustände und ermöglicht eine wesentliche Verbesserung der Patientencompliance.

## Patentansprüche

1. Pharmazeutische Formulierung mit verzögerter Freisetzung,
die 3-(3-Dimethylamino-1-ethyl-2-methyl-propyl)phenol oder ein pharmazeutisch annehmbares Salz davon
in einer Matrix mit verzögerter Wirkstofffreisetzung enthält,
wobei die Matrix 1 bis 80 Gew.-% eines oder mehrerer hydrophiler oder hydrophober Polymeren als pharmazeutisch annehmbaren Matrixbildner enthält und
in vitro die folgende Freisetzungsgeschwindigkeit aufweist, gemessen unter Anwendung der Ph. Eur. Paddle Method bei 75 U/min in einem Puffer (gemäß Ph. Eur.) bei einem pH-Wert von 6,8 bei 37 °C und unter UV-spektrometrischer Detektion:
3-35 Gew.-% (bezogen auf 100 Gew.-% Wirkstoff) 3-(3-Dimethylamino-1-ethyl-2-methyl-propyl)phenol nach 0,5 Stunden freigesetzt,
5-50 Gew.-% 3-(3-Dimethylamino-1-ethyl-2-methyl-propyl)phenol nach 1 Stunde freigesetzt,
10-75 Gew.-% 3-(3-Dimethylamino-1-ethyl-2-methyl-propyl)phenol nach 2 Stunden freigesetzt,
15-82 Gew.-% 3-(3-Dimethylamino-1-ethyl-2-methyl-propyl)phenol nach 3 Stunden freigesetzt,
30-97 Gew.-% 3-(3-Dimethylamino-1-ethyl-2-methyl-propyl)phenol nach 6 Stunden freigesetzt,
mehr als 50 Gew.-% 3-(3-Dimethylamino-1-ethyl-2-methylpropyl)phenol nach 12 Stunden freigesetzt,
mehr als 70 Gew.-% 3-(3-Dimethylamino-1-ethyl-2-methylpropyl)phenol nach 18 Stunden freigesetzt,
mehr als 80 Gew.-% 3-(3-Dimethylamino-1-ethyl-2-methylpropyl)phenol nach 24 Stunden freigesetzt.

2. Pharmazeutische Formulierung mit verzögerter Freisetzung, die 3-(3-Dimethylamino-1-ethyl-2-methyl-propyl)phenol oder ein pharmazeutisch annehmbares Salz davon in einer Matrix mit verzögerter Wirkstofffreisetzung enthält, wobei die Matrix 1 bis 80 Gew.-% eines oder mehrerer hydrophiler oder hydrophober Polymeren als pharmazeutisch annehmbaren Matrixbildner enthält und als pharmazeutisch annehmbaren Matrixbildner Celluloseether und/oder Celluloseester aufweist, der/die in einer 2 gew.-% wäßrigen Lösung bei 20 °C eine Viskosität von 3.000 bis 150.000 mPa·s aufweist.

3. Pharmazeutische Zusammensetzung nach einem der Ansprüche 1 bis 2, **dadurch gekennzeichnet, daß** sie als pharmazeutisch annehmbaren Matrixbildner Celluloseether und/oder Celluloseester aufweist, der/die in einer 2 gew.-% Lösung bei 20 °C eine Viskosität von 10.000 bis 150.000 mPa·s aufweist/aufweisen.

4. Pharmazeutische Zusammensetzung nach einem der Ansprüche 1 bis 3, **dadurch gekennzeichnet, daß** sie als pharmazeutisch annehmbaren Matrixbildner Celluloseether und/oder Celluloseester aufweist, der/die in einer 2 gew.-% Lösung bei 20 °C eine Viskosität von 50.000 bis 150.000 mPa·s aufweist/aufweisen.

5. Pharmazeutische Zusammensetzung nach einem der Ansprüche 1 bis 4, **dadurch gekennzeichnet, daß** sie als pharmazeutisch annehmbaren Matrixbildner mindestens eine Substanz enthält, die aus der Gruppe ausgewählt ist, die Hydroxypropylmethylcellulosen (HPMC), Hydroxyethylcellulosen, Hydroxypropylcellulosen (HPC), Methylcellulosen, Ethylcellulosen und Carboxymethylcellulosen umfaßt.

6. Pharmazeutische Zusammensetzung nach einem der Ansprüche 1 bis 5, **dadurch gekennzeichnet, daß** sie als pharmazeutisch annehmbaren Matrixbildner mindestens eine Substanz enthält, die aus der Gruppe ausgewählt ist, die Hydroxypropylmethylcellulosen, Hydroxyethylcellulosen und Hydroxypropylcellulosen umfaßt.

7. Pharmazeutische Zusammensetzung nach einem der Ansprüche 1 bis 6, **dadurch gekennzeichnet, daß** der Gehalt des verzögert freizusetzenden Wirkstoffs zwischen 0,5 und 85 Gew.-% und der Gehalt an pharmazeutisch akzeptablem Matrixbildner zwischen 8 und 40 Gew.-% liegt.

8. Pharmazeutische Zusammensetzung nach einem der Ansprüche 1 bis 7, **dadurch gekennzeichnet, daß** der Gehalt des verzögert freizusetzenden Wirkstoffs zwischen 3 und 70 Gew.-%, insbesondere zwischen 8 und 66 Gew.-%, und der Gehalt an pharmazeutisch akzeptablem Matrixbildner zwischen 10 und 35 Gew.-%, insbesondere zwischen 10 und 30 Gew.-%, liegt.

9. Pharmazeutische Zusammensetzung nach einem der Ansprüche 1 bis 8, **dadurch gekennzeichnet, daß** der Peak-Plasma-Level des Wirkstoffs in vivo nach 2 h bis 10 h, insbesondere nach 3,5 h bis 6 h erreicht wird.

10. Pharmazeutische Formulierung nach einem der Ansprüche 1 bis 9, **dadurch gekennzeichnet, daß** sie (+)-(1S,2S)-3-(3-Dimethylamino-1-ethyl-2-methyl-propyl)phenol oder ein pharmazeutisch annehmbares Salz davon enthält.

11. Pharmazeutische Formulierung nach einem der Ansprüche 1 bis 9, **dadurch gekennzeichnet, daß** sie (-)-(1R,2R)-3-(3-Dimethylamino-1-ethyl-2-methyl-propyl)phenol oder ein pharmazeutisch annehmbares Salz davon enthält.

12. Tablette für die 2-mal tägliche orale Verabreichung von 3-(3-Dimethylamino-1-ethyl-2-methyl-propyl)phenol, enthaltend eine pharmazeutische Formulierung nach einem der Ansprüche 1 bis 11.

## Claims

1. Pharmaceutical formulation with delayed release that contains 3-(3-dimethylamino-1-ethyl-2-methylpropyl)-phenol or a pharmaceutically acceptable salt thereof in a matrix with delayed release of active constituent, the matrix containing 1 to 80 wt.% of one or more hydrophilic or hydrophobic polymers as pharmaceutically acceptable matrix-forming agents, and exhibiting *in vitro* the following release rates, measured using the Ph. Eur. paddle method at 75 revs/min in a buffer (according to Ph. Eur.) at a pH value of 6.8 at 37°C and with UV spectrometric detection:
3-35 wt.% (referred to 100 wt.% of active constituent) of 3-(3-dimethylamino-1-ethyl-2-methylpropyl)phenol released after 30 minutes,
5-50 wt.% of 3-(3-dimethylamino-1-ethyl-2-methylpropyl)phenol released after 1 hour,
10-75 wt.% of 3-(3-dimethylamino-1-ethyl-2-methylpropyl)phenol released after 2 hours,
15-82 wt.% of 3-(3-dimethylamino-1-ethyl-2-methylpropyl)phenol released after 3 hours,
30-97 wt.% of 3-(3-dimethylamino-1-ethyl-2-methylpropyl)phenol released after 6 hours,
more than 50 wt.% of 3-(3-dimethylamino-1-ethyl-2-methylpropyl)phenol released after 12 hours,
more than 70 wt.% of 3-(3-dimethylamino-1-ethyl-2-methylpropyl)phenol released after 18 hours,
more than 80 wt.% of 3-(3-dimethylamino-1-ethyl-2-methylpropyl)phenol released after 24 hours.

2. Pharmaceutical formulation with delayed release that contains 3-(3-dimethylamino-1-ethyl-2-methylpropyl)-phenol or a pharmaceutically acceptable salt thereof in a matrix with delayed release of active constituent, the matrix containing 1 to 80 wt.% of one or more hydrophilic or hydrophobic polymers as pharmaceutically acceptable matrix-forming agent and comprises as pharmaceutically acceptable matrix-forming agent cellulose ethers and/or cellulose esters that, in a 2 wt.% aqueous solution at 20°C, have a viscosity of 3,000 to 150,000 mPa·s.

3. Pharmaceutical composition according to one of claims 1 and 2, **characterised in that** it contains as pharmaceutically acceptable matrix-forming agent cellulose ethers and/or cellulose esters that, in a 2 wt.% solution at 20°C, have a viscosity of 10,000 to 150,000 mPa·s.

4. Pharmaceutical composition according to one of claims 1 to 3, **characterised in that** it contains as pharmaceutically acceptable matrix-forming agent cellulose ethers and/or cellulose esters that, in a 2 wt.% solution at 20°C, have a viscosity of 50,000 to 150,000 mPa·s.

5. Pharmaceutical composition according to one of claims 1 to 4, **characterised in that** it contains as pharmaceutically acceptable matrix-forming agent at least one substance that is selected from the group comprising hydroxypropylmethyl celluloses (HPMC), hydroxyethyl celluloses, hydroxypropyl celluloses (HPC), methyl celluloses, ethyl celluloses and carboxymethyl celluloses.

6. Pharmaceutical composition according to one of claims 1 to 5, **characterised in that** it contains as pharmaceutically acceptable matrix-forming agent at least one substance that is selected from the group comprising hydroxypropylmethyl celluloses, hydroxyethyl celluloses and hydroxypropyl celluloses.

7. Pharmaceutical composition according to one of claims 1 to 6, **characterised in that** the content of the delayed release active constituent is between 0.5 and 85 wt.% and the content of pharmaceutically acceptable matrix-forming agent is between 8 and 40 wt.%.

8. Pharmaceutical composition according to one of claims 1 to 7, **characterised in that** the content of the delayed release active constituent is between 3 and 70 wt.%, in particular between 8 and 66 wt.%, and the content of pharmaceutically acceptable matrix-forming agent is between 10 and 35 wt.%, in particular between 10 and 30 wt.%.

9. Pharmaceutical composition according to one of claims 1 to 8, **characterised in that** the peak plasma level of the active constituent *in vivo* is reached after 2 to 10 hours, in particular after 3.5 to 6 hours.

10. Pharmaceutical formulation according to one of claims 1 to 9, **characterised in that** it contains (+)-(1S,2S)-3-(3-dimethylamino-1-ethyl-2-methylpropyl)phenol or a pharmaceutically acceptable salt thereof.

11. Pharmaceutical formulation according to one of claims 1 to 9, **characterised in that** it contains (-)-(1R,2R)-3-(3-dimethylamino-1-ethyl-2-methylpropyl)phenol or a pharmaceutically acceptable salt thereof.

12. Tablet for twice daily oral administration of 3-(3-dimethylamino-1-ethyl-2-methylpropyl)phenol, containing a pharmaceutical formulation according to one of claims 1 to 11.

## Revendications

1. Formulation pharmaceutique à libération retardée, qui contient du 3-(3-diméthylamino-1-éthyl-2-méthylpropyl)-phénol ou un sel pharmaceutiquement acceptable de ce composé dans une matrice à libération retardée de la substance active, la matrice contenant comme composants matriciels acceptables du point de vue pharmaceutique 1 à 80 % en poids d'un ou plusieurs polymères hydrophiles ou hydrophobes et présentant *in vitro* la vitesse de libération suivante, mesurée en utilisant la Paddle Method de la Pharmacopée Européenne à 75 tr/min dans un tampon (selon la Pharmacopée Européenne) à une valeur de pH de 6,8 à 37°C et avec détection par spectrométrie UV :
3 - 35 % en poids de 3-(3-diméthylamino-1-éthyl-2-méthylpropyl)-phénol (sur la base de 100 % en poids de substance active) libérés après 0,5 heure,
5 - 50 % en poids de 3-(3-diméthylamino-1-éthyl-2-méthylpropyl)-phénol libérés après 1 heure,
10 - 75 % en poids de 3-(3-diméthylamino-1-éthyl-2-méthylpropyl)-phénol libérés après 2 heures,
15 - 82 % en poids de 3-(3-diméthylamino-1-éthyl-2-méthylpropyl)-phénol libérés après 3 heures,
30 - 97 % en poids de 3-(3-diméthylamino-1-éthyl-2-méthylpropyl)-phénol libérés après 6 heures,
plus de 50 % en poids de 3-(3-diméthylamino-1-éthyl-2-méthylpropyl)-phénol libérés après 12 heures,
plus de 70 % en poids de 3-(3-diméthylamino-1-éthyl-2-méthylpropyl)-phénol libérés après 18 heures,
plus de 80 % en poids de 3-(3-diméthylamino-1-éthyl-2-méthylpropyl)-phénol libérés après 24 heures.

2. Formulation pharmaceutique à libération retardée, qui contient du 3-(3-diméthylamino-1-éthyl-2-méthylpropyl)-phénol ou un sel acceptable du point de vue pharmaceutique de ce composé dans une matrice à libération retardée de la substance active, la matrice contenant comme composant matriciel acceptable du point de vue pharmaceutique 1 à 80 % en poids d'un ou plusieurs polymères hydrophiles ou hydrophobes et présentant comme composant matriciel acceptable du point de vue pharmaceutique un éther de cellulose et/ou un ester de cellulose ayant, en solution aqueuse à 2 % en poids, une viscosité à 20°C de 3000 à 150 000 mPa.s.

3. Composition pharmaceutique suivant l'une des revendications 1 et 2, **caractérisée en ce qu'**elle présente comme composant matriciel acceptable du point de vue pharmaceutique un éther de cellulose et/ou un ester de cellulose présentant en solution aqueuse à 2 % en poids, une viscosité à 20°C de 10 000 à 150 000 mPa·s.

4. Composition pharmaceutique suivant l'une des revendications 1 à 3, **caractérisée en ce qu'**elle contient comme composant matriciel acceptable du point de vue pharmaceutique, un éther de cellulose et/ou un ester de cellulose présentant en solution aqueuse à 2 % en poids, une viscosité à 20°C de 50 000 à 150 000 mPa.s.

5. Composition pharmaceutique suivant l'une des revendications 1 à 4, **caractérisée en ce qu'**elle contient comme composant matriciel acceptable du point de vue pharmaceutique, au moins une substance qui est choisie dans le groupe comprenant des hydroxypropylméthylcelluloses (HPMC), des hydroxyéthylcelluloses, des hydroxypropylcelluloses (HPC), des méthylcelluloses, des éthylcelluloses et des carboxyméthylcelluloses.

6. Composition pharmaceutique suivant l'une des revendications 1 à 5, **caractérisée en ce qu'**elle contient comme composant matriciel acceptable du point de vue pharmaceutique, au moins une substance qui est choisie dans le groupe qui comprend des hydroxypropylméthylcelluloses, des hydroxyéthylcelluloses et des hydroxypropylcelluloses.

7. Composition pharmaceutique suivant l'une des revendications 1 à 6, **caractérisée en ce que** la teneur en substance active à libérer de façon retardée se situe entre 0,5 et 85 % en poids et la teneur en composant matriciel acceptable du point de vue pharmaceutique se situe entre 8 et 40 % en poids.

8. Composition pharmaceutique suivant l'une des revendications 1 à 7, **caractérisée en ce que** la teneur en substance active à libérer de façon retardée se situe entre 3 et 70 % en poids, notamment entre 8 et 66 % en poids et la teneur en composant matriciel acceptable du point de vue pharmaceutique se situe entre 10 et 35 % en poids, notamment entre 10 et 30 % en poids.

9. Composition pharmaceutique suivant l'une des revendications 1 à 8, **caractérisée en ce que** le pic du taux de substance active dans le plasma est atteint *in vivo* au bout de 2 à 10 heures, notamment au bout de 3,5 à 6 heures.

10. Formulation pharmaceutique suivant l'une des revendications 1 à 9, **caractérisée en ce qu'**elle contient du (+)-(1S,2S)-3-(3-diméthylamino-1-éthyl-2-méthylpropyl)-phénol ou un sel acceptable du point de vue pharmaceutique de ce composé.

11. Formulation pharmaceutique suivant l'une des revendications 1 à 9, **caractérisée en ce qu'**elle contient du (-)-(1R,2R)-3-(3-diméthylamino-1-éthyl-2-méthylpropyl)-phénol ou un sel acceptable du point de vue pharmaceutique de ce composé.

12. Comprimé destiné à l'administration orale, deux fois par jour, de 3-(3-diméthylamino-1-éthyl-2-méthylpropyl)-phénol, contenant une formulation pharmaceutique suivant l'une des revendications 1 à 11.
